(19) **Europäisches Patentamt**
**European Patent Office**
**Office européen des brevets**

(11) **EP 3 765 451 B1**

(12) **EUROPÄISCHE PATENTSCHRIFT**

(45) Veröffentlichungstag und Bekanntmachung des Hinweises auf die Patenterteilung:
**23.02.2022 Patentblatt 2022/08**

(21) Anmeldenummer: **19708552.5**

(22) Anmeldetag: **07.03.2019**

(51) Internationale Patentklassifikation (IPC):
**C07D 295/023** (2006.01)

(52) Gemeinsame Patentklassifikation (CPC):
**C07D 295/023**

(86) Internationale Anmeldenummer:
**PCT/EP2019/055676**

(87) Internationale Veröffentlichungsnummer:
**WO 2019/175008 (19.09.2019 Gazette 2019/38)**

(54) **VERFAHREN ZUM ENTFERNEN VON METHOXYETHANOL AUS EINEM METHOXYETHANOL UND MORPHOLIN ENTHALTENDEN GEMISCH**

METHOD FOR REMOVING METHOXYETHANOL FROM A METHOXYETHANOL AND MORPHOLIN-CONTAINING MIXTURE

PROCÉDÉ D'ÉLIMINATION DE MÉTHOXYÉTHANOL D'UN MÉLANGE CONTENANT DE LA MÉTHOXYÉTHANOL ET MORPHOLINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.03.2018 EP 18161945**

(43) Veröffentlichungstag der Anmeldung:
**20.01.2021 Patentblatt 2021/03**

(73) Patentinhaber: **BASF SE**
**67056 Ludwigshafen am Rhein (DE)**

(72) Erfinder:
• **HEIDEMANN, Thomas**
 **67056 Ludwigshafen (DE)**
• **KOCH, Eva**
 **67056 Ludwigshafen (DE)**
• **BECKER, Barbara**
 **67056 Ludwigshafen (DE)**
• **NIKOLIC, Lydia**
 **67056 Ludwigshafen (DE)**
• **SCHWABAUER, Inna**
 **67056 Ludwigshafen (DE)**
• **BICKELHAUPT, Jutta**
 **67056 Ludwigshafen (DE)**
• **GUETTLER, Antje**
 **67056 Ludwigshafen (DE)**
• **OEZKOZANOGLU, Claudia**
 **67056 Ludwigshafen (DE)**
• **HARNISCH, Uwe**
 **67056 Ludwigshafen (DE)**

(74) Vertreter: **BASF IP Association**
**BASF SE**
**GBI-C006**
**67056 Ludwigshafen (DE)**

(56) Entgegenhaltungen:
**WO-A1-2009/080507**

• **AMINI ET AL: "The effect of solution chemistry on the preparation of MgAl"2O"4 by hydrothermal-assisted sol-gel processing", MATERIALS RESEARCH BULLETIN, ELSEVIER, KIDLINGTON, GB, Bd. 42, Nr. 3, 22. Februar 2007 (2007-02-22), Seiten 563-570, XP005901091, ISSN: 0025-5408, DOI: 10.1016/J.MATERRESBULL.2006.06.011**

## Beschreibung

Die vorliegende Erfindung betrifft ein Verfahren zum Entfernen von Methoxyethanol aus einem Methoxyethanol und Morpholin enthaltenden Gemisch durch selektive Adsorption.

Die WO 2009/080507 A1 beschreibt ein Verfahren zur Herstellung eines Amins durch Umsetzung eines primären oder sekundären Aldehyds und/oder Ketons mit Wasserstoff und einer unter Ammoniak, primären Aminen und sekundären Aminen ausgewählten Stickstoffverbindung in Gegenwart eines Zirkondioxid, Kupfer und Nickel enthaltenden Katalysators. Als Beispiel wird ein Verfahren zur Herstellung von Morpholin beschrieben. Dabei wird Diethylenglycol dehydriert und der erhaltene Aldehyd reagiert mit Ammoniak unter Wasserabspaltung und anschließender Hydrierung zu einem Amin, welches zu Morpholin cyklisiert. In einer unerwünschten Parallelreaktion wird der Aldehyd jedoch decarbonyliert und es entsteht Methoxyethanol. Das Gemisch aus Reaktionsprodukten muss daher anschließend z. B. durch eine fraktionierte Rektifikation bei Vakuum, Normaldruck oder erhöhten Druck gereinigt werden.

Geeignete Aufarbeitungsverfahren sind z.B. in der EP-A-1 312 600 undEP-A-1 312 599 beschrieben. Leichtsieder- und Schwersiederfraktionen werden nacheinander destillativ abgetrennt und das erhaltende aminhaltige Gemisch mit Natronlauge extrahiert. Man erhält daraufhin eine wässrige, Natronlauge enthaltende Phase und eine zweite wässrige-organische Phase, die das Amin enthält. Anschließende Destillation der wässrig-organischen Phase führt zum wasserfreien Amin oder einem Amin/Wasser-Azeotrop, welches noch weiter gereinigt werden muss.

Die WO 2008/037589 A1 beschreibt ein Verfahren zur kontinuierlichen destillativen Auftrennung von Gemischen enthaltend Morpholin, Monoaminodiglykol, Ammoniak und Wasser.

CN 104262177 A beschreibt eine Methode für die Auftrennung eines Rohprodukts das aus Diglycolamin, Morpholin, Diglycol und Verunreinigung besteht mittels Säulenchromatographie.

Die bekannten Verfahren zur Aufreinigung der Methoxyethanol und Morpholin enthaltenden Gemische sind sehr aufwendig, mit einem erheblichen apparativen Aufwand bzw. einem deutlichen Energieaufwand verbunden. Ein Problem sind die sehr ähnlichen physikalischen Eigenschaften von Methoxyethanol und Morpholin. Diese erschweren die vollständige Abtrennung von Methoxyethanol vom Morpholin, was dazu führt dass ein Restgehalt von Methoxyethanol im Morpholin zurückbleibt. Dies kann zu Problemen hinsichtlich Spezifikation und Produktqualität führen.

Der vorliegenden Erfindung lag daher die Aufgabe zugrunde, ein Verfahren zum Entfernen von Methoxyethanol aus einem Methoxyethanol und Morpholin enthaltenden Gemisch anzugeben und einem oder mehreren Nachteilen des Standes der Technik abzuhelfen.

Es wurde nun gefunden, dass Methoxyethanol stärker als Morpholin an Mischoxide adsorbiert, die eine Spinellphase umfassen. Vermutlich ist Methoxyethanol in der Lage, über eine bidentate Bindung an Kristallitflächen der Spinellphase zu adsorbieren, während Morpholin aufgrund seiner Struktur und Konformation hierzu nicht in der Lage ist.

Die Erfindung betrifft daher ein Verfahren zum Entfernen von Methoxyethanol aus einem Methoxyethanol und Morpholin enthaltenden Gemisch durch selektive Adsorption von Methoxyethanol an ein Mischoxid, das eine Spinellphase umfasst.

Das Methoxyethanol und Morpholin enthaltende Gemisch ist in der Regel ein Reaktionsaustrag der Umsetzung von Diethylenglycol mit Ammoniak in Gegenwart eines A-minierungskatalysators. Das erfindungsgemäße Verfahren ist besonders zur Feinreinigung des Morpholins geeignet, das durch andere Verfahren vorgereinigt wurde. Typischerweise wird der primäre Reaktionsaustrag destillativ aufgereinigt. In einem ersten Schritt wird Ammoniak abgetrennt, in einem zweiten Schritt erfolgt die Abtrennung von Reaktionswasser und leichtsiedenden Nebenprodukten, in einem dritten Schritt erfolgt die Morpholinabtrennung und der vierte Schritt ist eine Morpholin-Reindestillation. Das bei der Reindestillation erhaltene Gemisch ist ein geeignetes Einsatzmaterial für das erfindungsgemäße Verfahren.

Der so erhaltene vorgereinigte Reaktionsaustrag enthält in der Regel neben Methoxyethanol und Morpholin wenigstens eine unter 1,2-Ethylendiamin, Methoxyethylmorpholin und Formylmorpholin ausgewählte Komponente. Das Methoxyethanol und Morpholin enthaltende Gemisch, das als Einsatzmaterial für das erfindungsgemäße Verfahren dient, enthält vorzugsweise weniger als 0,5 Gew.-%, insbesondere weniger als 0,3 Gew.-%, an von Methoxyethanol und Morpholin verschiedenen Komponenten. Es enthält vorzugsweise weniger als 0,5 Gew.-%, insbesondere weniger als 0,3 Gew.-%, Methoxyethanol, bezogen auf das Gesamtgewicht von Methoxyethanol und Morpholin. In der Regel enthält es 0,05 Gew.-% oder mehr Methoxyethanol.

Das erfindungsgemäß eingesetzte Mischoxid umfasst eine Spinellphase. Die Spinellphase ist vorzugsweise von einem Fremdoxid bzw. Trägeroxid begleitet. Das Fremdoxid weist eine von der Spinellstöchiometrie verschiedene Stöchiometrie und/oder eine von der Spinellstruktur verschiedene Struktur auf. Beispielsweise ist das Fremdoxid röntgenamorph. In der Regel sind kleine Kristallite der Spinellphase im Mischoxid dispergiert. Vorzugsweise beträgt die mittlere Kristallitgröße der Spinellphase 5 nm oder weniger. Die Bestimmung der mittleren Kristallitgröße gelingt z.B. durch Auswertung der Halbwertsbreite charakteristischer Beugungsreflexe im Pulverröngtendiffraktogramm nach der Scherrer-Gleichung oder nach der Rietveld-Methode.

Unter Spinellen im Sinne der vorliegenden Erfindung versteht man Metallmischoxide, in denen die Oxidionen die kubisch dichteste Kugelpackung einnehmen und die Elementarzelle 32 Sauerstoffionen enthält. Von den 64 Tetraederlücken in der Elementarzelle werden im Idealfall 8 Tetraederlücken von zweiwertigen Kationen A besetzt und von den 32 Oktaederlücken in der Elementarzelle werden im Idealfall 16 Oktaederlücken von dreiwertigen Kationen B besetzt, so dass die idealisierte Formel $AB_2O_4$ resultiert (Ullmann's Encyclopedia of Industrial Chemistry, 6. Auflage, 2003, Wiley-VCH Verlag GmbH & Co. KGaA, Weinheim, Band 26, Seiten 651-652).

In der Formel

$AB_2O_4$

steht A für ein zweiwertiges Kation, vorzugsweise Mg, Fe, Co, Ni, Mn, Zn oder Cd; und B für ein dreiwertiges oder vierwertiges Kation, vorzugsweise Al, Fe, Co, Cr, Ga, La oder Ti.

Unter Spinelle im Sinne der vorliegenden Erfindung fallen auch solche Metalloxide, die von der Idealformel abweichen und die durch die Phasen $A_{1-x}B_{2-x}O_4$ beschrieben werden können, wobei x die Werte von $0 < x \leq 1$ annehmen kann und gleichzeitig das molare Gesamtmetallverhältnis zu Sauerstoff 3 zu 4 beträgt.

Besonders bevorzugt weist die Spinellphase die idealisierte Formel $MgAl_2O_4$ auf.

Vorzugsweise umfasst das Mischoxid im Wesentlichen ausschließlich Oxide von Magnesium und Aluminium. Bevorzugt enthält das Mischoxid 20 bis 30 Gew.- % MgO und 80 bis 70 Gew.- % $Al_2O_3$, insbesondere 25 bis 27,5 Gew.-% MgO und 75 bis 82,5 Gew.-% $Al_2O_3$.

Vorzugsweise ist das Pulverröntgendiffraktogramm des Mischoxids gekennzeichnet durch Beugungsreflexe bei den Netzebenenabständen d [Ä] = 4,61 $\pm$ 0,1; 2,86 $\pm$ 0,1; 2,43 $\pm$ 0,1; 2,01 $\pm$ 0,1; 1,56 $\pm$ 0,1 und 1,42 $\pm$ 0,1. Aufgrund der kleinen Kristallitgröße können die Beugungsreflexe breit und schlecht aufgelöst sein.

Die Angabe der Röntgenbeugungsreflexe erfolgt in dieser Anmeldung in Form der von der Wellenlänge der verwendeten Röntgenstrahlung unabhängigen Netzebenenabstände d [Ä]. Die Wellenlänge $\lambda$, der zur Beugung verwendeten Röntgenstrahlung und der Beugungswinkel $\theta$ (als Beugungsreflexlage wird der Scheitelpunkt eines Reflexes in der $2\theta$-Auftragung verwendet) sind über die Bragg'sche Beziehung wie folgt miteinander verknüpft:

$$2\sin\theta = \lambda / d$$

wobei d der zum jeweiligen Beugungsreflex gehörende Netzebenenabstand der atomaren Raumanordnung ist.

Das Mischoxid liegt partikulär, z.B. in Form von Kugeln, Ringen, Tabletten, Extrudaten oder Splitt, vor. Die Partikel weisen z.B. eine mittlere Teilchengröße (in der Richtung der größten Raumausdehnung) von 1 bis 30 mm, vorzugsweise 5 bis 20 mm, auf. Beispielsweise kann ein Extrudat mit einem Durchmesser von 1,3 bis 1,5 mm und einer Länge von 5 bis 20 mm verwendet werden.

Die Herstellung der Mischoxide erfolgt nach an sich bekannten Verfahren, beispielsweise durch Co-Fällung aus den gemischten wässrigen Lösungen von Metallquellen, wie Metallnitraten, mit einer wässrigen Lösung eines Alkalimetallhydroxids und/oder Alkalimetallcarbonats und nachfolgender Calcinierung (W. Xu, Xi Liu, J. Ren, H. Liu, Y. Ma, Y. Wang, G. Lu, Microporous Mesoporous Mater. 2011, 142, Seiten 251-257). Der Calcinierungsschritt erfolgt im Allgemeinen bei Temperaturen im Bereich von 400 bis 1000 °C, vorzugsweise von 600 bis 900 °C. Das gewünschte Metallverhältnis wird durch das entsprechende Abmischen der Metallnitratlösungen eingestellt.

Die nach der Fällung erhaltenen Niederschläge werden zu den Spinellen weiterverarbeitet. Zunächst werden die Niederschläge gewaschen, wobei über die Menge und Temperatur des Waschwassers der Gehalt an Alkalimetall, zugeführt durch die als Fällungsreagenz verwendete Mineralbase, beeinflusst wird. Im Allgemeinen wird durch Verlängerung der Waschzeit oder Erhöhung der Temperatur der Gehalt an Alkalimetall verringert. Nach dem Waschen wird der Niederschlag getrocknet und gemahlen.

Der gewaschene und getrocknete Niederschlag kann mit Wasser angeteigt und extrudiert werden. Das Extrudat wird getrocknet und danach bei Temperaturen von 300 °C bis 800 °C, bevorzugt bei etwa 600 °C calciniert.

Alternativ kann das Mischoxid zu Formkörpern verpresst werden. Die Formgebung erfolgt vorzugsweise durch Tablettierung. Die Tablettierung ist ein Verfahren der Pressagglomeration. Dabei wird ein pulverförmiges oder vorab agglomeriertes Schüttgut in ein Presswerkzeug mit einer so genannten Matrize zwischen zwei Stempeln eingefüllt und durch einachsige Kompression verdichtet und zu einem festen Komprimat geformt. Die Tablettierung erfolgt z. B. auf so genannten Rundläuferpressen oder Exzenterpressen. Bei der Tablettierung können Tablettierhilfsmittel, wie Graphit oder Magnesiumstearat mitverwendet werden.

Zur selektiven Adsorption bringt man das Methoxyethanol und Morpholin enthaltende Gemisch mit dem Mischoxid in Kontakt, vorzugsweise indem man das Gemisch über eine Schüttung des Mischoxids leitet. Das Mischoxid liegt hierzu geeigneterweise in einer Festbettschüttung vor, die in einer Adsorptionssäule angeordnet ist, durch die der Stoffstrom geleitet wird. Die Adsorptionssäule ist vorzugsweise vertikal angeordnet und wird vom Stoffstrom in Richtung der Schwerkraft oder entgegen der Schwerkraft durchströmt. Die Ausdehnung des Festbetts in Strömungsrichtung beträgt vorzugsweise das 2- bis 15-fache des (längsten) Durchmessers des Festbetts. Es können auch mehrere hintereinandergeschaltete Adsorptionssäulen verwendet werden. Das von der Adsorptionssäule ablaufende Morpholin weist einen gegenüber dem aufgegebenen Gemisch einen verringerten Gehalt an Methoxyethanol auf.

Es wurde gefunden, dass die adsorptive Trennung von Methoxyethanol und Morpholin am Mischoxid in Abwesenheit von Wasser mit besserer Trennschärfe verläuft. Es ist daher bevorzugt, das Gemisch vor der selektiven Adsorption zu trocknen. In einer bevorzugten Ausführungsform trocknet man das Gemisch durch in Kontakt bringen mit einem Molekularsieb. Geeignet sind Molekularsiebe mit einer mittleren Porengröße von etwa 0,3 bis 0, 4 nm. So kann man das Gemisch über ein Festbett leiten, das stromaufwärts zur Strömungsrichtung des Gemisches in einer ersten Zone ein Molekularsieb und stromabwärts in einer zweiten Zone ein oben definiertes Mischoxid umfasst. Der Stoffstrom kommt in einer ersten Zone zuerst mit dem Molekularsieb in Kontakt, wobei bevorzugt Wasser adsorbiert wird. Größere sauerstoff- oder stickstoffhaltige Moleküle werden in dieser ersten Zone mit geringerer Präferenz adsorbiert. Erst in der anschließenden zweiten Zone wird Methoxyethanol bevorzugt vor Morpholin an das Mischoxid adsorbiert. Die beschriebene Ausführungsform, in der in einer ersten Zone bevorzugt Wasser entfernt wird, weist den Vorteil auf, dass - auch bei fortgeschrittener Sättigung des Mischoxids - keine Verdrängung von bereits adsorbiertem Methoxyethanol durch Wasser erfolgt.

Vor dem in Kontakt bringen mit dem Methoxyethanol und Morpholin enthaltenden Gemisch wird das Mischoxid vorzugsweise getrocknet. Das Trocknen kann durch Überleiten eines trockenen Inertgases, vorzugsweise Stickstoffgas, bei erhöhter Temperatur erfolgen. Geeignete Temperaturen sind 90 bis 200 °C, insbesondere 100 bis 150 °C. Das Trocknen des Mischoxids kann in mehreren Stufen bei ansteigender Temperatur erfolgen. Anschließend wird solange trockenes Inertgas über das Mischoxid geleitet, bis dieses abgekühlt ist. Wird eine strukturierte Schüttung von Molekularsieb und Mischoxid verwendet, wie vorstehend beschrieben, werden vorzugsweise beide Zonen durch Überleiten des trockenen Inertgases bei erhöhter Temperatur getrocknet.

Nach einer Betriebsdauer ist das Mischoxid gesättigt, d. h. seine Oberfläche ist mit Methoxyethanol belegt und die Adsorption wird zunehmend unselektiver bzw. beim Durchleiten des Stoffstroms findet keine weitere adsorptive Entfernung von Methoxyethanol aus dem Stoffstrom statt. Vorzugsweise wird das Mischoxid dann regeneriert. Zweckmäßigerweise kann man wenigstens zwei Adsorptionssäulen vorsehen, von denen sich eine erste Säule im Adsorptionszyklus befindet, während die andere Säule regeneriert wird. Ist das Mischoxid der ersten Säule gesättigt, wird der Stoffstrom umgeleitet und über die zweite Adsorptionssäule geleitet, so dass das Mischoxid in der ersten Säule regeneriert werden kann.

Die Regeneration des Mischoxids erfolgt geeigneterweise durch Behandlung mit Wasser. Dabei wird adsorbiertes Methoxyethanol vom Mischoxid gewaschen. Anschließend wird das Mischoxid getrocknet, wie oben beschrieben. Das Waschen des Mischoxids erfolgt vorzugsweise mit 5 bis 10 Waschfraktionen, wobei eine Waschfraktion dem Volumen des Adsorberbetts entspricht. Das Waschen wird bei Raumtemperatur durchgeführt, bevorzugt wird jedoch der Reaktor in den Waschfraktionen 3, 4 und 5 auf 80 °C geheizt und jeweils eine Stunde, gefüllt mit Wasser, stehen gelassen.

Um Morpholin-Verluste bzw. die Belastung des Waschwassers durch Morpholin, das am Mischoxid coadsorbiert ist, zu minimieren, ist es bevorzugt, coadsorbiertes Morpholin vor der Regeneration auszutreiben, vorzugsweise durch Überleiten eines Inertgases, wie Stickstoffgas, oder eines Wasserdampf-haltigen Inertgases, wie insbesondere angefeuchtetem Stickstoffgas. Das Austreiben des coadsorbierten Morpholins erfolgt vorzugsweise unter Erwärmen des Mischoxids auf erhöhte Temperatur von z. B. 50 bis 150 °C, insbesondere 50 bis 100 °C. Temperaturen bis 100 °C sind bevorzugt, da höhere Temperaturen zu Verfärbungen des zurückgewonnenen Morpholins führen können. Aus dem beladenden Inertgasstrom kann das Morpholin auskondensiert werden.

Die Erfindung wird durch die beigefügte Figur und die nachfolgenden Beispiele näher veranschaulicht.

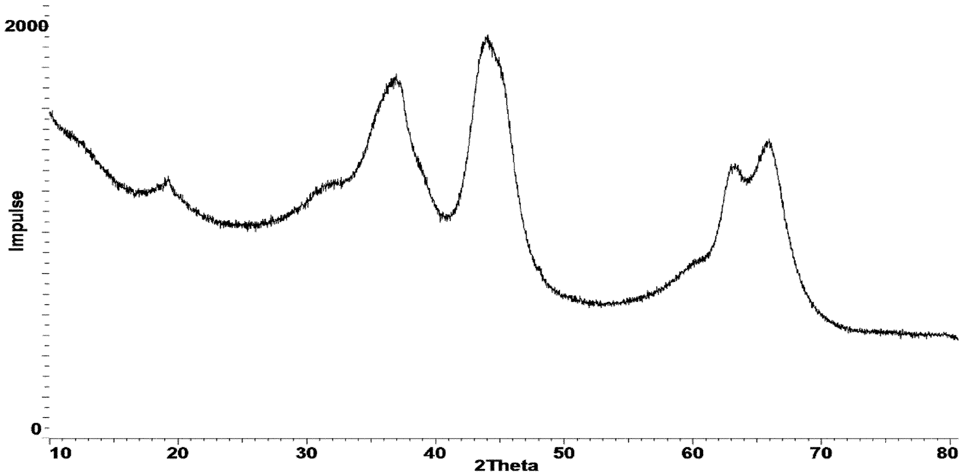

Fig. 1 zeigt das Pulverröntgendiffraktogramm des Mischoxids gemäß Beispiel 2.

Beispiele

Die XRD-Analysen wurden mit einem D8 Advance Serie 2 von der Firma Bruker/AXS unter Verwendung von CuK-alpha-Quelle (mit einer Wellenlänge von 0,154 nm bei 40 kV und 40 mA) durchgeführt. Die Messungen erfolgten über den Messbereich: 10-80° (2Theta), 0.02° Schritten mit 2,4 Sekunden/Schritt. Für die Ermittlung der mittleren Kristallitgrößen der einzelnen Phasen wurde die Strukturanalyse-Software TOPAS (Bruker AXS) verwendet.

Beispiel 1: Herstellung eines Spinell-haltigen Mischoxids, bestehend aus 25 % MgO und 75 % $Al_2O_3$.

Eine wässrige Lösung (1,95 L), die 628,23 g Magnesiumnitrat und 1889,2 g Aluminiumnitrat enthielt, wurde gleichzeitig in einen Rührgefäß in einem konstanten Strom mit einer 20 prozentigen wässrigen Natriumcarbonatlösung bei 80 °C so gefällt, dass der gemessene pH bei 5,5 gehalten wurde. Anschließend wurde der pH-Wert mit einer Natriumcarbonatlösung auf pH 7,8 eingestellt und die Reaktionslösung wurde für circa 30 min nachgerührt. Die erhaltene Suspension wurde filtriert und mit Wasser gewaschen, bis die Leitfähigkeit des Filtrats etwa 50 µS betrug und anschließend bei 100 °C für 16 Stunden getrocknet. Das Pulver wurde auf eine Korngröße von unter 500 µm gemahlen. Aus dem Pulver wurde ein Extrudat bei einem Druck von 80 bar mit 70 mL Wasser und einer Knetzeit von 70 min in Form von 1,5 mm langen Strängen hergestellt. Das erhaltene Extrudat wurde bei 120 °C 16 Stunden getrocknet und danach 1 Stunde bei 600 °C calciniert, wobei die Aufheizrate 2 °C/min betrug.

Beispiel 2

Der Spinell wurde wie in Beispiel 1 hergestellt, allerdings wurden die Magnesium- und Aluminiumnitratlösungen in einem anderen Verhältnis eingesetzt. Somit wurde ein Spinell mit der Zusammensetzung von 27,5 % MgO und 72,5 % $Al_2O_3$ erhalten.

Beispiel 3

Adsorption von Methoxethanol (MeOEtOH) an den Spinell aus Beispiel 1

Eine Laborsäule wurde mit einem Molekularsieb (100 mL) und dem Spinell (80 mL) bepackt, wobei das Molekularsieb vor den Spinell gepackt wurde. Danach wurden das Molekularsieb und der Adsorber in zwei Schritten getrocknet, dabei wurde zuerst Stickstoff (20 NL/h) für 20 Stunden bei 100 °C und anschließend für 6 Stunden bei 150 °C durchgeleitet. Über das trockene Adsorbermaterial wurde Morpholin geleitet, das etwa 0,1 Gew.-% Methoxyethanol enthielt. Die Adsorption wurde bei Raumtemperatur mit einer Flussrate von 10 g/h bis 15 g/h durchgeführt. Proben wurden regelmäßig mittels Gaschromatographie analysiert und als Durchbruch wurde eine Verringerung des Methoxyethanolgehalts um 50 % definiert.

Die Ergebnisse sind in der folgenden Tabelle dargestellt.

| Probe | Methoxyethanol Fl.-% | Kumulierte Belastung Adsorber $kg_{MeOEtOH}/t_{Adsorber}$ | Abnahme Methoxyethanol [%] |
|---|---|---|---|
| 1 | 0,0310 | 3,48 | 71,52 |
| 2 | 0,0194 | 8,05 | 82,17 |
| 3 | 0,0240 | 11,10 | 78,02 |
| 4 | 0,0433 | 16,43 | 60,23 |
| 5 | 0,0601 | 20,01 | 44,86 |
| 6 | 0,0764 | 24,91 | 29,93 |

Die Kapazität des Adsorbers betrug etwa 17 $kg_{MeOEtOh}/t_{adsorber}$.

Beispiel 4

Adsorption von Methoxyethanol an den Spinell aus Beispiel 2

Die Adsorptionsversuche wurden analog zu Beispiel 3 durchgeführt. Die Ergebnisse sind in der folgenden Tabelle dargestellt.

| Probe | Methoxyethanol Fl.-% | Kumulierte Belastung Adsorber $kg_{MeOEtOH}/t_{Adsorber}$ | Abnahme Methoxyethanol [%] |
|---|---|---|---|
| 1 | 0,008 | 3,60 | 92,66 |
| 2 | 0,000 | 5,85 | 100,00 |
| 3 | 0,006 | 9,50 | 94,50 |
| 4 | 0,061 | 14,15 | 44,04 |
| 5 | 0,087 | 17,30 | 20,18 |

Die Kapazität des Adsorbers betrug etwa 14 $kg_{MeOEtOh}/t_{adsorber}$.

Beispiel 5

Regeneration des Spinells aus Beispiel 1

Der Spinell aus Beispiel 1 wurde durch Waschen mit Wasser bei Raumtemperatur regeneriert. Dazu wurden 10 Waschfraktionen verwendet, wobei eine Waschfraktion einem Bettvolumen entspricht und die Flussrate 400 g/h betrug. Jede Waschfraktion wurde mittels Gaschromatographie analysiert und das Mischoxid wurde anschließend mit Stickstoff getrocknet (20 NL/h; 2 d bei 80°C, 2 h bei 100 °, 2 h bei 120 °C, 6 h bei 150 °C).

Die Ergebnisse der Gaschromatographie (Flächen-%) sind in der nachstehenden Tabelle dargestellt. Dabei wurde deutlich dass das Waschwasser noch erhebliche Mengen an Morpholin enthielt.

| Fraktion | Morpholin-Gehalt Fl.-% |
|----------|------------------------|
| 1 | 30,48 |
| 2 | 7,46 |
| 3 | 0,49 |
| 4 | 0,07 |
| 5 | 0,02 |
| 6 | 0,02 |
| 7 | 0,02 |
| 8 | 0,01 |
| 9 | 0,01 |
| 10 | 0,01 |

Beispiel 6

Regeneration des Spinells aus Beispiel 2

Der Spinell wurde nach unterschiedlichen Verfahren regeneriert.

Bei der ersten Regeneration wurde die Säule auf 50 °C bis 80 °C geheizt und es wurde trockener Stickstoff durchgeleitet. Anschließend wurde der beladene Stickstoff über einen 5 °C kalten Kondensor geleitet, wo sich Morpholin abschied. Danach wurde der Adsorber mit 5 Waschfraktionen Wasser gewaschen und mit Stickstoff getrocknet. Das abgeschiedene Morpholin, sowie die Waschfraktionen wurden mittels Gaschromatographie analysiert.

Die zweite Regenration wurde analog zur Ersten durchgeführt, mit dem Unterschied, dass die Säule auf 50 °C bis 150 °C geheizt wurde. Am Kondensor abgeschiedenes Morpholin und die Waschfraktionen wurden mittels Gaschromatographie analysiert. Aus den Daten wurde der Morpholin-Verlust abgeschätzt.

Bei der dritten Regeneration wurde die Säule auf 50 °C bis 100 °C geheizt und nach dem Durchleiten des trockenen Stickstoffes folgte eine Behandlung mit Stickstoff, der mit einer Waschflasche angefeuchtet wurde, welche Wasser bei Raumtemperatur enthielt. Die nachfolgende Regeneration wurde analog zur zweiten Regeneration durchgeführt, wobei abgeschiedenes Morpholin und die Waschfraktionen mittels Gaschromatographie analysiert wurden.

Die vierte Regeneration wurde analog zur Dritten durchgeführt, mit dem Unterschied, dass der Stickstoff mit 90 °C warmen Wasser angefeuchtet wurde.

| Regeneration | Morpholin-Verlust % |
|--------------|---------------------|
| 1 | 0,34 |
| 2 | 0,18 |
| 3 | 0,21 |
| 4 | 0,11 |

**Patentansprüche**

1. Verfahren zum Entfernen von Methoxyethanol aus einem Methoxyethanol und Morpholin enthaltenden Gemisch durch selektive Adsorption von Methoxyethanol an ein Mischoxid, das eine Spinellphase umfasst.

2. Verfahren nach Anspruch 1, **dadurch gekennzeichnet, dass** die Spinellphase die Formel

$$AB_2O_4$$

aufweist, worin

A für ein zweiwertiges Kation steht, vorzugsweise Mg, Fe, Co, Ni, Mn, Zn oder Cd; und

B für ein dreiwertiges oder vierwertiges Kation steht, vorzugsweise Al, Fe, Co, Cr, Ga, La oder Ti.

3. Verfahren nach Anspruch 2, **dadurch gekennzeichnet, dass** die Spinellphase die Formel $MgAl_2O_4$ aufweist.

4. Verfahren nach Anspruch 3, **dadurch gekennzeichnet, dass** das Mischoxid 20 bis 30 Gew.- % MgO und 80 bis 70 Gew.- % $Al_2O_3$, insbesondere 25 bis 27,5 Gew.-% MgO und 75 bis 82,5 Gew.-% $Al_2O_3$ enthält.

5. Verfahren nach einem der vorhergehenden Ansprüche, **dadurch gekennzeichnet, dass** man das Gemisch über eine Schüttung des Mischoxids leitet.

6. Verfahren nach einem der vorhergehenden Ansprüche, **dadurch gekennzeichnet, dass** das Gemisch außerdem wenigstens eine unter 1,2-Ethylendiamin, Methoxyethylmorpholin und Formylmorpholin ausgewählte Komponente enthält.

7. Verfahren nach einem der vorhergehenden Ansprüche, **dadurch gekennzeichnet, dass** man das Gemisch vor der selektiven Adsorption trocknet.

8. Verfahren nach Anspruch 7, **dadurch gekennzeichnet, dass** man das Gemisch durch in Kontakt bringen mit einem Molekularsieb trocknet.

9. Verfahren nach einem der vorhergehenden Ansprüche, **dadurch gekennzeichnet, dass** man das Mischoxid durch Behandlung mit Wasser regeneriert.

10. Verfahren nach Anspruch 9, **dadurch gekennzeichnet, dass** man coadsorbiertes Morpholin vor der Regeneration des Mischoxids austreibt.

11. Verfahren nach Anspruch 10, **dadurch gekennzeichnet, dass** man coadsorbiertes Morpholin durch Überleiten eines Inertgases oder eines Wasserdampf-haltigen Inertgases austreibt.

12. Verfahren nach Anspruch 11, **dadurch gekennzeichnet, dass** man das ausgetriebene coadsorbierte Morpholin aus dem Inertgases oder Wasserdampf-haltigen Inertgas auskondensiert.

**Claims**

1. A method for removing methoxyethanol from a mixture comprising methoxyethanol and morpholine by selective adsorption of methoxyethanol onto a mixed oxide comprising a spinel phase.

2. The method according to claim 1, wherein the spinel phase has the formula

$AB_2O_4$

in which

A is a divalent cation, preferably Mg, Fe, Co, Ni, Mn, Zn or Cd; and

B is a trivalent or tetravalent cation, preferably Al, Fe, Co, Cr, Ga, La or Ti.

3. The method according to claim 2, wherein the spinel phase has the formula $MgAl_2O_4$.

4. The method according to claim 3, wherein the mixed oxide comprises 20 to 30% by weight MgO and 80 to 70% by weight $Al_2O_3$, especially 25 to 27.5% by weight MgO and 75 to 82.5% by weight $Al_2O_3$.

5. The method according to any of the preceding claims, wherein the mixture is passed over a bed of the mixed oxide.

6. The method according to any of the preceding claims, wherein the mixture comprises in addition at least one component selected from 1,2-ethylenediamine, methoxyethylmorpholine and formylmorpholine.

7. The method according to any of the preceding claims, wherein the mixture is dried prior to the selective adsorption.

8. The method according to claim 7, wherein the mixture is dried by bringing it into contact with a molecular sieve.

9. The method according to any of the preceding claims, wherein the mixed oxide is regenerated by treatment with water.

10. The method according to claim 9, wherein coadsorbed morpholine is desorbed prior to the regeneration of the mixed oxide.

11. The method according to claim 10, wherein coadsorbed morpholine is desorbed by passing over an inert gas or an inert gas containing steam.

12. The method according to claim 11, wherein the desorbed coadsorbed morpholine is condensed out from the inert gas or inert gas containing steam.

**Revendications**

1. Procédé pour l'élimination de méthoxyéthanol à partir d'un mélange contenant du méthoxyéthanol et de la morpholine par adsorption sélective de méthoxyéthanol sur un oxyde mixte qui comprend une phase de spinelle.

2. Procédé selon la revendication 1, **caractérisé en ce que** la phase de spinelle présente la formule

$$AB_2O_4$$

dans laquelle
A représente un cation divalent, de préférence Mg, Fe, Co, Ni, Mn, Zn ou Cd ; et
B représente un cation trivalent ou tétravalent, de préférence Al, Fe, Co, Cr, Ga, La ou Ti.

3. Procédé selon la revendication 2, **caractérisé en ce que** la phase de spinelle présente la formule $MgAl_2O_4$.

4. Procédé selon la revendication 3, **caractérisé en ce que** l'oxyde mixte contient 20 à 30 % en poids de MgO et 80 à 70 % en poids d'$Al_2O_3$, en particulier 25 à 27,5 % en poids de MgO et 75 à 82,5 % en poids d'$Al_2O_3$.

5. Procédé selon l'une quelconque des revendications précédentes, **caractérisé en ce qu'**on conduit le mélange à travers un tas de l'oxyde mixte.

6. Procédé selon l'une quelconque des revendications précédentes, **caractérisé en ce que** le mélange contient en outre au moins un composant choisi parmi la 1,2-éthylènediamine, la méthoxyéthylmorpholine et la formylmorpholine.

7. Procédé selon l'une quelconque des revendications précédentes, **caractérisé en ce qu'**on sèche le mélange avant l'adsorption sélective.

8. Procédé selon la revendication 7, **caractérisé en ce que** le mélange est séché par mise en contact avec un tamis moléculaire.

9. Procédé selon l'une quelconque des revendications précédentes, **caractérisé en ce qu'**on régénère l'oxyde mixte par traitement avec de l'eau.

10. Procédé selon la revendication 9, **caractérisé en ce qu'**on chasse la morpholine coadsorbée avant la régénération de l'oxyde mixte.

11. Procédé selon la revendication 10, **caractérisé en ce qu'**on chasse la morpholine coadsorbée par passage d'un gaz inerte ou d'un gaz inerte contenant de la vapeur d'eau.

12. Procédé selon la revendication 11, **caractérisé en ce qu'**on condense, depuis le gaz inerte ou le gaz inerte contenant de la vapeur d'eau, la morpholine coadsorbée chassée.

EP 3 765 451 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009080507 A1
- EP 1312600 A
- EP 1312599 A
- WO 2008037589 A1
- CN 104262177 A

**In der Beschreibung aufgeführte Nicht-Patentliteratur**

- Ullmann's Encyclopedia of Industrial Chemistry. Wiley-VCH Verlag GmbH & Co. KGaA, 2003, vol. 26, 651-652
- **W. XU ; XI LIU ; J. REN ; H. LIU ; Y. MA ; Y. WANG ; G. LU.** *Microporous Mesoporous Mater,* 2011, vol. 142, 251-257